# EUROPEAN PATENT APPLICATION

(11) **EP 1 719 526 A2**
(43) Date of publication of application: **08.11.2006**
(21) Application number: 06075955.2
(22) Date of filing: 29.11.2000
(51) Int. Cl.: A61K 45/06, A61K 31/7076, A61K 31/397, A61P 7/02

(54) **Pharmaceutical combinations comprising a P2T receptor antagonist and another anti-thrombotic agent**

(30) Priority: 01.12.1999 SE 9904377
(62) Divisional of application: 00982033.3
(71) Applicant: Astra Zeneca AB, S-151 85 Södertalje (SE)
(72) Inventor: Dixon, John, Loughborough Leicestershire LE11 5RH (GB); Humphries, Robert, Loughborough Leicestershire LE11 5RH (GB); Jarvis, Gavin, Cambridge CB2 2EQ (GB); Kirk, Ian, Loughborough Leicestershire LE11 5RH (GB)
(74) Representative: Mercer, Christopher Paul

(57) **Abstract**

The present invention provides novel pharmaceutical combinations and their use in anti-thrombotic therapy.

## Description

### FIELD OF THE INVENTION

The present invention relates to pharmaceutical combinations comprising a P_{*2T*} receptor antagonist and another anti-thrombotic agent and to their use in the treatment and prevention of thrombosis.

### BACKGROUND OF THE INVENTION

Increased understanding of the mechanisms underlying thrombosis and of interventions therein has led to a polypharmacological anti-thrombotic approach utilising appropriate combinations of anti-platelet, anti-coagulant and fibrinolytic agents. Examples of anti-thrombotic compounds used include anti-platelet agents such as aspirin, clopidogrel, ticlopidine, GPIIb/IIIa antagonists; anti-coagulants such as thrombin inhibitors, warfarin, heparin and low molecular weight heparins; and fibrinolytic agents including but not limited to, streptokinase, tissue plasminogen activator (tPA) and tenecteplase.

Most patients with acute myocardial infarction are currently treated using either a thrombolytic agent or intervention treatment with percutaneous coronary angioplasty (PTCA). It has been shown that the use of both these methods result in an increase in the number of patients achieving acceptable coronary artery patency at 90 minutes, and that the better the flow in the affected coronary artery, the greater the survival.

However, even the most effective thrombolytic agents with adjunctive aspirin and heparin treatment are only moderately effective in achieving coronary artery patency with normal blood flow (assessed as TIMI grade 3) in about 50% of cases. In addition, in the acute setting where immediate effect is paramount, slow-acting agents leave a window where the patient is not protected from thrombosis. For example, clopidogrel inhibits ADP-induced platelet aggregation and, like the earlier analogue, ticlopidine, has shown clinical efficacy in arterial thrombosis. However, both agents produce incomplete, slow to develop inhibition of the ADP response, properties which are far from ideal in acute therapy, such as prevention of stent occlusion, although increasing use of a loading dose has been a recent advance.

Another short-coming of existing anti-thrombotic agents, and combinations thereof, is that the optimal pharmacodynamic risk:benefit (anti-thrombotic:anti-haemostatic) relationship has not yet been achieved.

Thus there is a need for more effective anti-thrombotic therapy.

Recently it has been shown that P_{*2T*} (also known as P2Y_{ADP} or P2T_{AC}) receptor antagonists offer significant improvements over other anti-thrombotic agents. The P_{*2T*} receptor is primarily involved in mediating platelet aggregation/activation and is a G-protein coupled receptor. The pharmacological characteristics of this receptor have been described, for example, in the references by Humphries et al., Br. J. Pharmacology (1994), 113, 1057-1063, and Fagura et al., Br. J. Pharmacology (1998) 124, 157-164.

International Patent Applications WO 92/17488 and WO 94/18216 disclose novel P_{2T} receptor antagonists and thereof, including compounds of formula (I) (see below). Compound A (a compound of formula (I) wherein R, is 3,3,3-trifluoropropyl and R₂ is 2-(methylthio)ethyl) is disclosed in WO 94/18216. Compound B (a compound of formula (I) wherein R, is propyl and R, is hydrogen) is disclosed in WO 92/17488.

Both compound A and compound B may be used in any condition where platelet activation or aggregation is involved. The compounds may thus act as anti-thrombotic agents and are useful in the treatment or prophylaxis of unstable angina, thromboembolic stroke and peripheral vascular disease. They may also be used in the treatment and prophylaxis of the sequalae of thrombotic complications from angioplasty, thrombolysis, endarterectomy, coronary and vascular graft surgery, renal dialysis and cardio-pulmonary bypass. In addition, they can be used in the treatment and prophylaxis of disseminated intravascular coagulation, deep vein thrombosis, pre-eclampsia, tissue salvage following surgical or accidental trauma, vasculitis, arteritis, thrombocythaemia, ischemia and migraine.

### DISCLOSURE OF THE INVENTION

The inventors of the present invention have surprisingly found that administration of a combination of a P_{*2T*} receptor antagonist or a pharmaceutically acceptable derivative thereof, and another anti-thrombotic agent or a pharmaceutically acceptable derivative thereof, offers a significant improvement over other anti-thrombotic treatments.

Accordingly, the combined administration of a P_{*2T*} receptor antagonist or a pharmaceutically acceptable derivative thereof and another anti-thrombotic agent or a pharmaceutically acceptable derivative thereof, can be used in the treatment and prevention of thrombosis, particularly in the treatment of the thrombotic complications of atherosclerotic disease and interventions therein.

According to a first aspect of the invention there is provided a kit of parts comprising:
(a) a P_{*2T*} receptor antagonist or a pharmaceutically acceptable derivative thereof (component a); and
(b) another anti-thrombotic agent or a pharmaceutically acceptable derivative thereof (component b);
   where components (a) and (b) are each provided in a form (which may be the same or different) that is suitable for administration in conjunction with each other.

Pharmaceutically acceptable derivatives of a P_{*2T*} receptor antagonist and other anti-thrombotic agent include salts (e.g. pharmaceutically acceptable non-toxic organic or inorganic acid addition salts (such as a salt of hydrochloric, hydrobromic, nitric, sulphuric or acetic acid)), solvates and solvates of salts.

If more than one formulation comprising component (a) or component (b) is present, for example in order to provide for repeat dosing, such formulations may be the same, or may be different in terms of the dosage, chemical composition and/or physical form.

Preferably, the P_{*2T*} receptor antagonist is the compound of formula (I): 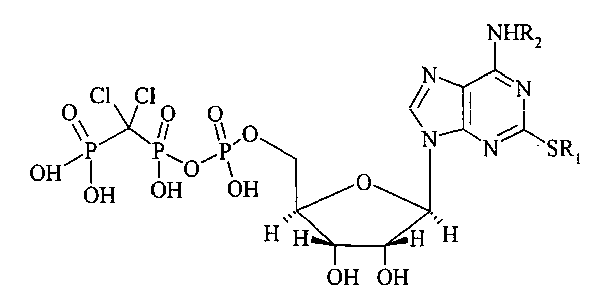 wherein:
either R, is 3,3,3-trifluoropropyl and R₂ is 2-(methylthio)ethyl
or R, is propyl and R₂ is hydrogen;
or a pharmaceutically acceptable derivative thereof.

More preferably, the P_{*2T*} receptor antagonist is compound A (where R, is 3,3,3-trifluoropropyl and R₂ is 2-(methylthio)ethyl as disclosed in WO 94/18216).

Preferably component (b) is selected from anti-platelet agents, anti-coagulant agents, fibrinolytic agents, and any combination thereof.

More preferably, component (b) is selected from the group consisting of aspirin, clopidogrel, ticlopidine, a GPIIb/IIIa antagonist, direct thrombin inhibitors, prodrugs of direct thrombin inhibitors, warfarin, heparin, low molecular weight heparins, streptokinase, tissue plasminogen activator, tenecteplase and any combination thereof. Examples of direct thrombin inhibitors include melagatran (WO 94/29336). Prodrugs of melagatran include those described in WO 97/23499, and particularly include Example 17 of that application. Example 17 of WO 97/23499 is H 376, which is EtO₂C-CH₂-(R)Cgl-Aze-Pab-OH, wherein Cgl is cyclohexylglycinyl, Aze is (S)-azetidine-2-carbonyl and Pab is para-amidinobenzylamino and the OH replaces one of the amidino hydrogens in Pab.

In accordance with the invention, the P_{*2T*} receptor antagonist, other anti-thrombotic agent, and derivatives of either, may be administered orally, intravenously, subcutaneously, buccally, rectally, dermally, nasally, tracheally, bronchially, topically, or via inhalation into the lung. Preferred modes of delivery are systemic. For the P_{*2T*} receptor antagonist and derivatives thereof, preferred modes of administration are parenteral; more preferably intravenous. For the other anti-thrombotic agent and derivatives thereof, preferred modes of administration are oral or, in the case of unfractionated or low molecular weight heparins, certain direct thrombin inhibitors and fibrinolytic agents, intravenous or subcutaneous.

The sequence in which the formulations comprising the P_{*2T*} receptor antagonist and the other anti-thrombotic agent may be administered (i.e. whether, and at what point, sequential, separate and/or simultaneous administration takes place) may be determined by the physician or skilled person. For example, the sequence may depend upon many factors, such as whether, at any time during the course or period of treatment, one or other of the formulations cannot be administered to the person for practical reasons (e.g. the person is unconscious and thus unable to take an oral formulation).

Respective formulations comprising component (a) and/or component (b) may be administered, sequentially, separately and/or simultaneously, over the course of treating the relevant condition, which condition may be acute or chronic. Preferably, the two formulations are administered (optionally repeatedly) sufficiently closely in time for there to be a beneficial effect for the patient, that is greater, over the course of the treating the relevant condition, than if either of the two formulations are administered (optionally repeatedly) alone, in the absence of the other formulation, over the same course of treatment. Determination of whether a combination provides a greater beneficial effect in respect of, and over the course of treatment of a particular condition, will depend upon the condition to be treated or prevented, but may be achieved routinely by the skilled person.

Alternatively, one or other of the two component formulations may be administered (optionally repeatedly) prior to, after, and/or at the same time as, administration with the other component. Individual doses of a P_{*2T*} receptor antagonist and other anti-thrombotic agent may be used within 48 hours (e.g. 24 hours) of each other.

In the therapeutic treatment of mammals, and especially humans, the P_{*2T*} receptor antagonist, other anti-thrombotic agent, and derivatives of either, may be administered alone, but will generally be administered as a pharmaceutical formulation in admixture with a pharmaceutically acceptable adjuvant, diluent or carrier, which should be selected with due regard to the intended route of administration and standard pharmaceutical practice.

In accordance with the invention, the kit of parts may be used in medical therapy, suitably in the treatment of thrombosis. The treatment of thrombosis will be understood by those skilled in the art to include the treatment and prevention of thrombotic complications of atherosclerotic disease and interventions therein, such as fibrinolysis, endarterectomy or percutaneous transluminal coronary revascularisation (PTCR), including, but not limited to, percutaneous transluminal coronary angioplasty (PTCA) with or without stenting. Thrombotic complications of atherosclerotic disease include, but are not limited to, acute coronary syndrome (encompassing acute myocardial infarction with or without ST elevation and unstable angina) and thrombotic stroke.

A further aspect of the invention provides a method of treating thrombosis (for example thrombotic complications of atherosclerotic disease and interventions therein, such as fibrinolysis, endarterectomy or percutaneous transluminal coronary revascularisation (PTCR), including, but not limited to, percutaneous transluminal coronary angioplasty (PTCA) with or without stenting) which comprises using a kit of parts for administering a therapeutically effective amount of a P 2T receptor and another anti-thrombotic agent to a person suffering from or susceptible to such a disorder.

For avoidance of doubt the term "treatment" includes therapeutic and/or prophylactic treatment.

Preferably component component (a) is administered parenterally prior to surgery and component (b) is administered orally following that surgery.

According to another aspect of the invention, there is provided a method of making a kit of parts as defined herein, which comprises bringing a component (a) into association with a component (b) thus rendering the two components suitable for administration in conjunction with each other. By bringing the two components into association with each other, we include that components (a) and (b) may be:
i) packaged presented and purchased as separate formulations which are subsequently used in conjunction in combination therapy; or
ii) packaged and presented together as separate components of a combination pack for use in conjunction with each other in combination therapy.

The present invention still further provides a kit of parts comprising:
(1) components (a) and (b) as defined herein; together with
(2) instructions to use the components in conjunction with each other.

The invention further provides the use of a P_{*2T*} receptor antagonist, or a pharmaceutically acceptable derivative thereof, in the manufacture of a kit of parts for the treatment of thrombosis.

Components (a) and (b) as described herein may also be co-formulated as a combined preparation (i.e. presented as a single formulation including a P_{*2T*} receptor antagonist and other anti-thrombotic agent).

Thus, a further aspect of the invention provides a pharmaceutical formulation comprising:
(a) a P_{*2T*} receptor antagonist or a pharmaceutically acceptable derivative thereof; and
(b) another anti-thrombotic agent or a pharmaceutically acceptable derivative thereof; in admixture with a pharmaceutically acceptable adjuvant, diluent or carrier.

Preferably component (a) is a compound of formula (I) as defined above, or a pharmaceutically acceptable derivative thereof. Preferably component (b) is selected from anti-platelet agents, anti-coagulant agents, fibrinolytic agents, and any combination thereof. More preferably, component (b) is selected from the group consisting of aspirin, clopidogrel, ticlopidine, a GPIIb/IIIa antagonist, direct thrombin inhibitors, prodrugs of direct thrombin inhibitors, warfarin, heparin, low molecular weight heparins, tissue plasminogen activator, tenecteplase, and any combination thereof.

The present invention provides a pharmaceutical formulation comprising:
(a) a P_{*2T*} receptor antagonist or a pharmaceutically acceptable derivative thereof; and
(b) another anti-thrombotic agent or a pharmaceutically acceptable derivative thereof; in admixture with a pharmaceutically acceptable adjuvant, diluent or carrier;
   for use in medical therapy, suitably in the treatment of thrombosis.

The invention further provides a method of treating thrombosis which comprises administering a therapeutically effective amount of a pharmaceutical formulation comprising:
(a) a P_{*2T*} receptor antagonist or a pharmaceutically acceptable derivative thereof; and
(b) another anti-thrombotic agent or a pharmaceutically acceptable derivative thereof; in admixture with a pharmaceutically acceptable adjuvant, diluent or carrier;
   to a person suffering from or susceptible to such a disorder.

In another aspect of the present invention, there is provided a process for the preparation of a pharmaceutical formulation which comprises mixing a P_{*2T*} receptor antagonist with another anti-thrombotic agent.

The invention further provides the use of a pharmaceutical formulation as hereinbefore defined in the manufacture of a medicament for the treatment of thrombosis.

Another aspect of the invention involves the use of:
(a) a pharmaceutical formulation comprising a P_{*2T*} receptor antagonist or a pharmaceutically acceptable derivative thereof, in admixture with a pharmaceutically acceptable adjuvant, diluent or carrier; and
(b) a pharmaceutical formulation comprising another anti-thrombotic agent or a pharmaceutically acceptable derivative thereof, in admixture with a pharmaceutically acceptable adjuvant, diluent or carrier,
   in therapy, suitably in the treatment of thrombosis.

A further aspect of the invention provides a method of treating thrombosis which comprises administering:
a) a pharmaceutical formulation comprising a P_{*2T*} receptor antagonist or a pharmaceutically acceptable derivative thereof, in admixture with a pharmaceutically acceptable adjuvant, diluent or carrier, and
b) a pharmaceutical formulation comprising another anti-thrombotic agent or a pharmaceutically acceptable derivative thereof, in admixture with a pharmaceutically acceptable adjuvant, diluent or carrier,
   to a person suffering from or susceptible to such a disorder.

In another aspect of the present invention, there is provided the use of a P_{*2T*} receptor antagonist, or a pharmaceutically acceptable derivative thereof, in the manufacture of a medicament to be used in combination with another anti-thrombotic agent in the treatment of thrombosis. Preferably the P_{*2T*}receptor antagonist is a compound of formula (I), or a pharmaceutically acceptable derivative thereof.

Suitable formulations for administering a P_{*2T*} receptor antagonist are known in the art, and include those known from WO 92/17488 and WO 94/18216.

Suitable formulations for administering other anti-thrombotic agent are described in the literature, for example, when the other anti-thrombotic agent is melagatran, or a prodrug of melagatran, suitable formulations include those described in *inter alia* WO 94/29336, WO 96/14084, WO 96/16671, WO 97/23499, WO 97/39770, WO 97/45138, WO 98/16252, WO 99/27912, WO 99/27913, WO 00/13672 and WO 00/12043. Otherwise, the preparation of suitable formulations may be achieved by the skilled person using routine techniques.

Suitable doses of the P_{*2T*} receptor antagonist, the other anti-thrombotic agent, and derivatives of either can be determined by the medical practitioner or other skilled person, and will depend on the severity of the condition, and on the person to be treated, as well as the compound(s) which is/are employed. Respective doses are discussed in the prior art documents disclosing P_{*2T*} receptor antagonists and other anti-thrombotic agents that are mentioned hereinbefore.

In the case of a compound of formula (I), suitable doses of active compound in the therapeutic and/or prophylactic treatment of mammalian, especially human, patients include those which give a mean plasma concentration of up to 5 µmol/L, for example in the range 0.001 to 5 µmol/L over the course of treatment of the relevant condition. In any event, the physician, or the skilled person, will be able to determine the actual dosage which will be most suitable for an individual person, which is likely to vary with the condition that is to be treated, as well as the age, weight, sex and response of the particular person to be treated. The above-mentioned dosages are exemplary of the average case. There can, of course, be individual instances where higher or lower dosage ranges are merited, and such are within the scope of this invention.

The pharmaceutical formulation of the invention may, and indeed will usually, contain various other ingredients known in the art, for example preservatives, stabilising agents, viscosity-regulating agents, emulsifying agents or buffering agents. Thus the pharmaceutical formulation of the invention will typically comprise a total amount of (a) the P_{*2T*} receptor antagonist and (b) another anti-thrombotic agent (the active ingredients) in the range from 0.05 to 99 %w (per cent by weight), more preferably in the range from 0.10 to 70 %w, and even more preferably in the range from 0.10 to 50 %w, all percentages by weight being based on total formulation.

### EXAMPLES

The invention is illustrated, but in no way limited, by the following examples, either utilising compound A (a compound of formula (I) wherein R₁ is 3,3,3-trifluoropropyl and R, is 2-(methylthio)ethyl) or, in Example 3, using data obtained with the close structural analogue, compound B (a compound of formula (I) wherein R, is propyl and R, is hydrogen, synthesised at AstraZeneca R & D, Chamwood, Humphries et al (1995), Br J Pharmacol., 115; 1110-1116).

### Example 1

### Canine Coronary Thrombosis Model - compound A and aspirin/heparin

Compound A was used in combination with aspirin and unfractionated heparin in a dog model of coronary artery thrombosis to determine whether addition of a P_{*2T*}-receptor antagonist to these standard anti-platelet and anti-coagulant agents could improve coronary artery patency after thrombolysis with tPA. All animals were treated with both aspirin 325 mg and unfractionated heparin 80 U/kg then 17 U/kg/h. The test group (n = 10) was treated with compound A (4 µg/kg/min iv) from 10 min prior to tPA until end of protocol (2 h post reperfusion). The placebo group (n = 10) received only a saline infusion iv from 10 min prior to tPA until end of protocol (2 h post reperfusion).

The results of the experiments are evident in tables 1 and 2.

Coronary artery blood flow following successful thrombolysis with tPA was significantly better maintained in a group of animals receiving compound A in addition to aspirin and heparin than in a group receiving saline, aspirin and heparin (table 1).

**Table 1. Effects on coronary thrombolysis by tPA**

| **Parameter** | **Saline** | **Compound A** |
|---|---|---|
| Baseline blood flow (ml/min) | 65.3 ± 7.5 | 62.3 ± 8.5 ns |
| Time to occlusion (min) | 55.5 ± 14.0 | 62.3 ± 14.7ns |
| Reperfusion rate | 100% | 100%ns |
| Time to reflow (min) | 21.5 ± 2.9 | 20 ± 6.1ns |
| Reflow duration (min) | 75.0 ± 39.9 | 119.7 ± 0.7* |
| Cyclic flow variation | 50% | 0%* |
| Reocclusion | 60% | 0%* |

| | | |
|---|---|---|
| * P<0.05 | | |

Infarct size was also reduced significantly (P < 0.05) in animals receiving compound A (table 2). These results suggest that significant additional clinical benefit will be attained when a P_{*2T*} antagonist is combined with a fibrinolytic agent and standard anti-platelet and anti-coagulant therapy.

**Table 2. Infarct size reduction**

| | Saline | Compound A | |
|---|---|---|---|
| Area at risk (cm²) | 48.7 ±6.9 | 49.9 ± 8.4 | Ns |
| Infarct size (cm²) | 9.3 ± 4.4 | 4.7 ± 4.7 | P = 0.034 |

### Example 2

### Human blood in vitro - compound A and clopidogrel

Compound A (500 nM final concentration) was added to blood from healthy human volunteers receiving clopidogrei (Sanofi-Winthrop, 75 mg/day for 11 days). ADP-induced platelet aggregation (+/- compound A) was measured using whole blood impedance aggregometry.

Clopidogrel alone resulted in slowly developing, incomplete inhibition of the ADP response (Table 3). Compound A added *in vitro* produced complete or near complete inhibition of the response to low to intermediate concentrations of ADP (up to 30 µM) both before and during administration of clopidogrel (data for ADP 10 µM shown in Table 3) while substantial inhibition of the response to the highest concentration of ADP used (300 µM) required a combination of both compound A and clopidogrel.

**Table 3: Effect of oral clopidogrel (75 mg/day) on ADP (10 and 300 µM)-induced platelet aggregation measured in blood from healthy human volunteers ex vivo (+/compound A (500 nM) added in vitro)**

| Duration of clopidogrel administration (days) | Aggregation (ohms) (mean ± SD, n = 7 - 8 except where indicated) | | | |
|---|---|---|---|---|
| | ADP 10 µM | | ADP 300 µM | |
| | - compound A | + compound A | - compound A | + compound A |
| 0 | 14.9 ± 1.9 | 0.5 ±0.7 | Not measured | 6.5 ± 3.4 |
| 1 | 13.8 ± 2.3 | 0.4 ± 0.7 | Not measured | 4.2 ± 2.6 |
| 2 | 11.8 ± 3.4 | 0.4 ± 0.6 | Not measured | 2.9 ± 2.3 |
| 3 | 10.2±4.5 | 0.6±0.7 | 13.9 ⁽ⁿ⁼¹⁾ | 2.7 ± 2.1 |
| 11 | 8.2±4.4 | 0.6±0.8 | 11.4 ± 5.2 ⁽ⁿ⁼³⁾ | 2.8 ± 2.8 |

### Example 3

P-selectin expression on the platelet membrane surface plays an important role in platelet-leukocyte-conjugate formation and there is increasing evidence that such interactions play an important role in both acute thrombosis and in the inflammatory aetiology of progressive atheroscerosis. The effect of a P_{*2T*}-receptor antagonist (compound B) on ADP (10 µM)-induced platelet P-selectin expression was investigated in human washed platelets. The effect of compound B (10 nM) was compared with that of the GPIIb/IIIa antagonist, GR144053 (10 µM, Foster et al (1993) Thromb Haemostas;69(6):559, synthesized in AstraZeneca R & D, Charnwood). These concentrations are 4 (compound B)- and 600 (GR144053)-fold higher than the respective IC₅₀ values for inhibition of ADP-induced platelet aggregation in this system. The results are summarised in Table 4.

**Table 4: Effect of compound B and GR144053 alone or in combination on ADP (10 µM)-induced P-selectin expression in human washed platelets**

| Conditions | P-selectin expression (% positive cells) Mean ± se (n = 3) |
|---|---|
| Control | 13.1 ± 3.3 |
| + GR144053 (10 µM) | 17.7 ± 1.7 |
| + compound B (10 nM) | 4.9 ± 2.6 |
| + GR14405 (10 µM) + compound B (10 nM) | 7.5 ± 1.6 |

The P_{*2T*}-receptor antagonist, at a concentration consistent with known effects on ADP-induced platelet aggregation, substantially inhibits ADP-induced P-selectin expression in the absence or presence of the GPIIb/IIIa antagonist. In contrast, the GPIIb/III antagonist alone had no effect on P-selectin expression at a concentration considerably in excess of that which would completely inhibit platelet aggregation. These results suggest the potential for combination therapy with GPIIb/IIIa antagonists and P_{*2T*}-receptor antagonists, wherein the broad-spectrum anti-aggregatory effect of the former class is complemented by the additional effect of the latter on other aspects of platelet activation, such as pro-inflammatory P-selectin expression.

### Abbreviations

ADP = adenosine diphosphate
GPIIb/IIIa antagonist = glycoprotein IIb/IIIa antagonist
PTCR = percutaneous transluminal coronary revascularisation
PTCA = percutaneous transluminal coronary angioplasty
TIMI = thrombolysis in myocardial infarction
tPA = tissue plasminogen activator

## Claims

1. A kit of parts comprising:
(a) a P_{*2T*} receptor antagonist or a pharmaceutically acceptable derivative thereof (component a); and
(b) another anti-thrombotic agent or a pharmaceutically acceptable derivative thereof (component b);
where components (a) and (b) are each provided in a form (which may be the same or different) that is suitable for administration in conjunction with each other.

2. A kit of parts according to claim 1, wherein component (a) is a compound of formula (1): 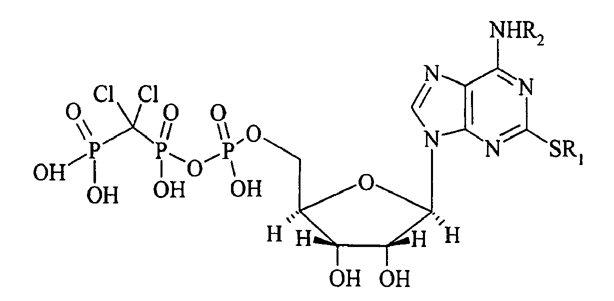 wherein:
either R₁ is 3,3,3-trifluoropropyl and R₂ is 2-(methylthio)ethyl
or R₁ is propyl and R₂ is hydrogen,
or a pharmaceutically acceptable derivative thereof.

3. A kit of parts according to claim 1 or 2, wherein the anti-thrombotic agent is selected from anti-platelet agents, anti-coagulant agents, fibrinolytic agents, and any combination thereof.

4. A kit of parts according to any one of claims 1 to 3, wherein the anti-thrombotic agent is selected from the group consisting of aspirin, clopidogrel, ticlopidine, a GPIIb/IIIa antagonist, direct thrombin inhibitors, prodrugs of direct thrombin inhibitors, warfarin, heparin, low molecular weight heparins, tissue plasminogen activator, tenecteplase, and any combination thereof.

5. A kit of parts according to any one of claims 1 to 4, wherein the anti-thrombotic agent is a direct thrombin inhibitor and/or a prodrug of a direct thrombin inhibitor.

6. A kit of parts as claimed in claim 5, wherein the thrombin inhibitor is melagatran.

7. A kit of parts as claimed in claim 5, wherein the prodrug of melagatran is EtO₂C-CH₂₋(R)Cgl-Aze-Pab-OH

8. A kit of parts according to any one of claims 1 to 7, wherein components (a) and (b) are suitable for sequential, separate and/or simultaneous administration.

9. A kit of parts according to any one of claims 1 to 7, for use in medical therapy.

10. A kit of parts according to any one of claims 1 to 7, for use in the treatment of thrombosis.

11. A method of treating thrombosis which comprises using a kit of parts according to any one of claims 1 to 7, for administering a therapeutically effective amount of a P_{*2T*} receptor and another anti-thrombotic agent to a person suffering from or susceptible to such a disorder.

12. A method according to claim 11, wherein component (a) is administered parenterally prior to surgery and component (b) is administered orally following that surgery.

13. The use of a P_{*2T*}receptor antagonist according to any one of claims 1 to 12, or a pharmaceutically acceptable derivative thereof, in the manufacture of a kit of parts for the treatment of thrombosis.

14. A pharmaceutical formulation comprising:
(a) a P_{*2T*} receptor antagonist or a pharmaceutically acceptable derivative thereof; and
(b) another anti-thrombotic agent or a pharmaceutically acceptable derivative thereof; in admixture with a pharmaceutically acceptable adjuvant, diluent or carrier.

15. A pharmaceutical formulation according to claim 14, wherein the P_{*2T*} receptor antagonist is a compound of formula (I) as defined in claim 2, or a pharmaceutically acceptable derivative thereof.

16. A pharmaceutical formulation according to claim 14 or 15, wherein the anti-thrombotic agent is selected from anti-platelet agents, anti-coagulant agents, fibrinolytic agents, and any combination thereof.

17. A pharmaceutical formulation according to any one of claims 14 to 16, wherein the anti-thrombotic agent is selected from the group consisting of aspirin, clopidogrel, ticlopidine, a GPIIb/IIIa antagonist, direct thrombin inhibitors, prodrugs of direct thrombin inhibitors, warfarin, heparin, low molecular weight heparins, tissue plasminogen activator, tenecteplase, and any combination thereof.

18. A pharmaceutical formulation according to any one of claims 14 to 17, wherein the anti-thrombotic agent is a direct thrombin inhibitor and/or a prodrug of a direct thrombin inhibitor.

19. A pharmaceutical formulation according to claim 18, wherein the thrombin inhibitor is melagatran.

20. A pharmaceutical formulation according to claim 18, wherein the prodrug of melagatran is EtO₂C-CH₂-(R)Cgl-Aze-Pab-OH

21. A pharmaceutical formulation according to any one of claims 14 to 20 for use in medical therapy.

22. A pharmaceutical formulation according to any one of claims 14 to 20 for use in the treatment of thrombosis.

23. The use of a pharmaceutical formulation according to any one of claims 14 to 20 in the manufacture of a medicament for the treatment of thrombosis.

24. A method of treating thrombosis which comprises administering a therapeutically effective amount of a pharmaceutical formulation according to any one of claims 14 to 20 to a person suffering from or susceptible to such a disorder.

25. A process for the preparation of a pharmaceutical formulation according to any one of claims 14 to 20 which comprises mixing a P_{*2T*} receptor antagonist with another anti-thrombotic agent.

26. The use of:
(a) a pharmaceutical formulation comprising a P_{*2T*} receptor antagonist or a pharmaceutically acceptable derivative thereof, in admixture with a pharmaceutically acceptable adjuvant, diluent or carrier; and
(b) a pharmaceutical formulation comprising another anti-thrombotic agent or a pharmaceutically acceptable derivative thereof, in admixture with a pharmaceutically acceptable adjuvant, diluent or carrier,
in therapy.

27. The use of:
(a) a pharmaceutical formulation comprising a P_{*2T*} receptor antagonist or a pharmaceutically acceptable derivative thereof, in admixture with a pharmaceutically acceptable adjuvant, diluent or carrier; and
(b) a pharmaceutical formulation comprising another anti-thrombotic agent or a pharmaceutically acceptable derivative thereof, in admixture with a pharmaceutically acceptable adjuvant, diluent or carrier,
in the treatment of thrombosis

28. The use of a pharmaceutical formulation according to claims 26 or 27, wherein the P_{*2T*} receptor antagonist is a compound of formula (I) as defined in claim 2.

29. A method of treating thrombosis which comprises administering to a person suffering from, or susceptible to such a condition:
(a) a pharmaceutical formulation comprising a P_{*2T*} receptor antagonist, or a pharmaceutically acceptable derivative thereof, in admixture with a pharmaceutically acceptable adjuvant, diluent or carrier, and
(b) a pharmaceutical formulation comprising another anti-thrombotic agent or a pharmaceutically acceptable derivative thereof, in admixture with a pharmaceutically acceptable adjuvant, diluent or carrier.

30. A method according to claim 29, wherein the P_{*2T*} receptor antagonist is a compound of formula (I) as defined in claim 2.

31. A method according to claim 29 or 30, wherein the anti-thrombotic agent is selected from anti-platelet agents, anti-coagulant agents, and any combination thereof.

32. A method according to any one of claims 29 to 31, wherein the anti-thrombotic agent is selected from the group consisting of aspirin, clopidogrel, ticlopidine, a GPIIb/IIIa antagonist, direct thrombin inhibitors, prodrugs of direct thrombin inhibitors, warfarin, heparin, low molecular weight heparins, tissue plasminogen activator, tenecteplase, and any combination thereof.

33. A method according to any one of claims 29 to 32, wherein the anti-thrombotic agent is a direct thrombin inhibitor and/or a prodrug of a direct thrombin inhibitor.

34. A method according to claim 33, wherein the thrombin inhibitor is melagatran.

35. A method according to claim 33, wherein the prodrug of melagatran is EtO₂C-CH₂₋(R)Cgl-Aze-Pab-OH

36. A method according to any one of claims 29 to 35, wherein component (a) is a parenteral formulation and component (b) is an oral formulation.

37. A method according to any one of claims 29 to 36, wherein component (a) is administered parenterally prior to surgery and component (b) is administered orally following that surgery.

38. The use of a P_{*2T*} receptor antagonist, or a pharmaceutically acceptable derivative thereof, in the manufacture of a medicament to be used in combination with another anti-thrombotic agent in the treatment of thrombosis

39. The use of a compound of formula (I) as defined in claim 2, or a pharmaceutically acceptable derivative thereof, in the manufacture of a medicament to be used in combination with another anti-thrombotic agent in the treatment of thrombosis.
